Europäisches Patentamt

European Patent Office

Office européen des brevets

(11) Veröffentlichungsnummer: **0 331 620 B1**

(12) **EUROPÄISCHE PATENTSCHRIFT**

(45) Veröffentlichungstag der Patentschrift: **11.08.93**

(51) Int. Cl.5: **A61K 31/70**

(21) Anmeldenummer: **89730051.3**

(22) Anmeldetag: **01.03.89**

(54) **Mittel zur Behandlung des Parkinson-Syndroms.**

(30) Priorität: **01.03.88 DE 3807003**
**15.03.88 DE 3809024**

(43) Veröffentlichungstag der Anmeldung:
**06.09.89 Patentblatt 89/36**

(45) Bekanntmachung des Hinweises auf die
Patenterteilung:
**11.08.93 Patentblatt 93/32**

(84) Benannte Vertragsstaaten:
**AT BE CH DE ES FR GB GR IT LI LU NL SE**

(56) Entgegenhaltungen:

**JOURNAL OF NEURO-VISCERAL RELATIONS,
Band 31, Suppl. IX, 1969, Seiten 297-308; W.
BIRKMAYER: "The Importance of Monoamine
Metabolism for the Pathology of the Extrapyramidal System"**

**ARCHIV FÜR PSYCHIATRIE UND ZEIT-
SCHRIFT F.D. GES. NEUROLOGIE, Band 210,
Nr. 1, 1967, Seiten 29-35; W. BIRKMAYER et
al.: "Weitere experimentelle Untersuchungen
über den Catecholaminstoffwechsel bei extrapyramidalen Erkrankungen (Parkinson-
und Chorea-Syndrom)**

(73) Patentinhaber: **Birkmayer, Walther, Prof. Dr.
Schwarzspanierstrasse 15
A-1090 Wien(AT)**

Patentinhaber: **Birkmayer, Jörg, Prof.-Dr.
Schwarzspanierstrasse 15
A-1090 Wien(AT)**

(72) Erfinder: **Birkmayer, Walther, Prof.Dr.
Schwarzspanierstrasse 15
A-1090 Wien(AT)**
Erfinder: **Birkmayer, Jörg, Dr.
Schwarzspanierstrasse 15
A-1090 Wien(AT)**
Erfinder: **Horowski, Reinhard, Dr.
Sigismundkorso 52
W-1000 Berlin 28(DE)**
Erfinder: **Wachtel, Helmut, Dr.
Suarezstrasse 22
W-1000 Berlin 19(DE)**
Erfinder: **Löschmann, Peter-Andreas
Württembergallee 8
W-1000 Berlin 19(DE)**

BIOCHEMICAL AND BIOPHYSICAL RESE-
ARCH COMMUNICATIONS, Band 135, Nr. 1,
26. Februar 1986, Seiten 269-275, Academic
Press, Inc.; R. RAMSAY et al.: "Inhibition of
mitochondrial NADH dehydrogenase by pyridine derivatives and its possible relation to
experimental and idiopathic parkinsonism"

THE LANCET, Band 1 (8369), 1984, Seite 167;
J. BLAIR et al.: "Aetiology of Parkinson's
disease"

JOURNAL OF NEUROCHEMISTRY, Band 48,
Nr. 3, 1987, Seiten 876-882, International Society for Neurochemistry; M. GRISHAM et al.:
"Neuromelanogenic and Cytotoxic Properties of Canine Brainstem Peroxidase"

JOURNAL OF NEUROCHEMISTRY, Band 49,
Nr. 1, 1987, Seiten 1-8, International Society
for Neurochemistry; T.P. SINGER et al.:
"Biochemical Events in the Development of
Parkinsonism Induced by
1-Methyl-4-Phenyl-1,2,3,6-Tetrahydropyridine-
"

ANN. CLIN. LAB. SCI., Band 19, nr. 1, Januar-
Februar 1989, Seiten 38-43, Institute for Chemical Science, Inc.; W. BIRKMAYER et al.:
"Nicotinamidadenindinucleotide (NADH):
The New Approach in the Therapy of Parkinson's Disease"

(74) Vertreter: **von Hellfeld, Axel, Dr. Dipl.-Phys. et
al
Wuesthoff & Wuesthoff Patent- und Rechtsanwälte Schweigerstrasse 2
W-8000 München 90 (DE)**

**Beschreibung**

Gegenstand der Erfindung ist die Verwendung von Nicotinamid-adenin-dinucleotid sowohl in nicht reduzierter (NAD) wie in reduzierter Form (NADH) oder eines Salzes davon ausgenommen in einer Kombination mit Tyrosin zur Herstellung eines zur Behandlung des Parkinson-Syndroms geeigneten Arzneimittels.

Die Parkinson'sche Krankheit beruht auf einer gestörten dopaminergen Neurotransmission in den Basalganglien, im allgemeinen infolge eines zunehmenden Untergangs der dopaminergen Neuronen zunächst in der Substantia nigra, im Verlauf der Krankheit auch in anderen Arealen. In fortgeschrittenen Fällen wird auch ein zunehmender Noradrenalin-Mangel (z.B. im Locus caeruleus) feststellbar. Schon in frühen Stadien der Erkrankung findet man auch einen sehr deutlichen Abfall der Tyrosin-Hydroxylase-Aktivität, die interessanterweise auch in anderen Systemen und Organen (z.B. im Nebennierenmark) stark vermindert ist (s. Tab. 3 aus Birkmayer und Riederer, Die Parkinson-Krankheit, 2. Aufl., S. 34, Springer Verlag Wien, 1985).

Tabelle 3

| Tyrosinhydroxylase in verschiedenen Gehirnarealen beim Morbus Parkinson | | | |
|---|---|---|---|
| Gehirnareale | | Kontrollen | M. Parkinson |
| N. caudatus | (15) | 27,8 ± 2,3 | 3.5 ± 1,0 (6)* |
| Putamen | (5) | 16,2 ± 5,9 | 1,2 ± 0,4 (6)* |
| S. nigra | (4) | 19,4 ± 6,2 | 4,9 ± 1,8 (4)* |
| L. caeruleus | (4) | 3,3 ± 0,1 | 2,0 ± 0,6 (2) |
| N. ruber | (5) | 5,7 ± 1,9 | 2,1 ± 1,4 (3) |
| Raphe + R.F. | (4) | 0,9 ± 0,6 | 1,5 ± 0,4 (5) |
| Hypothalamus | (5) | 3,1 ± 1,0 | 1,5 ± 0,3 (3) |
| C. mamillare | (5) | 0,6 ± 0,4 | 0,5 ± 0,9 (2) |
| N. accumbens | (5) | 2,0 ± 0,7 | 2,7 ± 2,2 (3) |
| Nebenniere (Medulla) | (5) | 186,2 ± 5,5 | 49,7 ± 12,4 (4)* |
| Anzahl der Patienten in Klammern. | | | |
| Mittelwerte ± sem (nmol Dopa/g-Gewebe.Stunde). | | | |
| * p < 0,01. | | | |

Bei der konventionellen Therapie wird die fehlende oder verminderte dopaminerge Aktivität durch die Dopamin-Vorstufe L-DOPA ersetzt; bei Nachlassen dieser Wirkung kann die L-DOPA-Wirkung durch spezifische Hemmung des Dopamin-abbauenden Enzyms MAO-B nochmals gesteigert werden.

Diese Behandlungsstrategien führten jedoch, obwohl durch sie die Lebenserwartung der Patienten verbessert wurde, nach Jahren der Anwendung häufig zu schweren und nicht vorhersagbaren Schwankungen der Beweglichkeit (Fluktuationen, on-off-Phänomene), die die Lebensqualität der Patienten empfindlich beeinträchtigen. Diese können teilweise durch rechtzeitigen Einsatz von postsynaptischen Agonisten wie Lisurid vermieden werden oder zumindest vermindert werden.

Für die plötzliche Einschränkung der Beweglichkeit wurde von Birkmayer und Riederer diskutiert, daß ihr eine vorübergehende Enzymerschöpfung der Tyrosin-Hydroxylase (Birkmayer und Riederer, Die Parkinson-Krankheit, 2. Aufl., S. 81, Springer Verlag Wien, 1985) zugrunde liegt. In der Tat läßt sich auch biochemisch durch einen Teil der Standard-Therapien eine vermehrte Belastung der Tyrosin-Hydroxylase postulieren.

Es ist anzunehmen, daß unter normalen Bedingungen dieses Schlüssel-Enzym der Dopamin- (und Noradrenalin-) Synthese ausreichend induziert und mit Co-Faktoren versorgt ist. Beim Parkinsonismus kann jedoch ein Fehler der Programmierung der Tyrosin-Hydroxylase-Synthese ursächlich beteiligt sein.

Klinische Untersuchungen haben überraschenderweise ergeben, daß auch bei Patienten mit fortgeschrittenem Parkinsonismus und schweren Schwankungen der Beweglichkeit die Gabe des Enzym-Kofaktors Nicotinamid-adenin-dinucleotid eine geradezu dramatische Besserung der Motorik verursacht, ohne daß ein Zusatz von p-Tyrosin erforderlich ist, wie es in Archiv für Psychiatric f.d.ges. Neurologie, Band 210, 5.29 bis 35, 1967, beschrieben war.

Die klinischen Ergebnisse sind am Beispiel von 3 Parkinson-Kranken in der nachfolgenden Tabelle dargestellt.

| Alter (Jahre) | | Disability |
|---|---|---|
| 73 | Krankheitsdauer 9 Jahre | |
| | ohne Therapie | 75 % |
| | 25 mg NADH in 250 ml NaCl 0,9 % i.v. tägl. v. 30.11. - 19.12.87 (stationär) | 20 % |
| | Madopar(R) 62,5 2 x tägl. v. 19.12.87 - 4.1.88 (zu Hause) | 50 - 60 % |
| | Bei Zugabe von 50 mg NADH langsam i.v. einmal wöchentl. zur Basistherapie 2 x 62,5 Madopar (zu Hause) (Beobachtung bis 25.1.88) | 30 % |
| 59 | Krankheitsdauer 8 Jahre | |
| | bisherige Therapie 3 x 1/2 Sinemet(R) - 3 x 1 | 60 % |
| | 2.11. - 14.11.87 3 x 1/2 Sinemet(R) Jumex 1 Tablette Jatrosom(R) 1 Tablette + 50 mg NADH täglich | 30 % im "On" |
| | Festsetzung der NADH Inf. 1 x wöchentl. | 20 % "Off"-Zeiten wurden kürzer |

| Alter (Jahre) | Disability |
|---|---|
| 62 Krankheitsdauer 8 Jahre | |
| 19.2.86 | 40 % |
| unter 3 x 125 Madopar® | |
| 2 Tab Jumex® | |
| Saroten R 2 mg | |
| bis 2.11.86 Verschlechterung auf | 60 % |
| unter 2 Amp. Oxyferiscorbone 2 Amp./d | |
| Madopar® 3 x 1/2 | |
| Artane® 2 x 2 mg | |
| nach 3 Wochen Verbesserung auf | 30 % |
| 28.8.87 Prostata Operation | |
| 20.9.87 unter 3 x 250 mg Madopar® | |
| 2 x 1 Tab Jumex® | |
| 250 mg Tryptophan | |
| zusätzlich 50 mg NADH 2 x wöchentl.i.v. | 20 % (on)/60 % (off) |
| ohne NADH | 40 % (on)/80 % (off) |
| 10.2.88 2 x 250 mg Madopar® | |
| 2 x 1 Jumex® | |
| 10 mg Saroten® | |
| u. 2x wöchentl. 50 mg NADH i.v. | 20 - 30 % (on) |
| | 60 - 70 % (off) |

Die Daten der Tabelle zeigen am Beispiel von NADH, daß die Einnahme des Enzym-Kofaktors oder eines Salzes davon zu einer deutlichen Verbesserung der Disability führt. Dies wird sowohl in der on- wie auch in der off-Phase festgestellt und wird sowohl bei gleichzeitiger Gabe von Standard-Präparaten wie auch ohne gleichzeitige Gabe von bekannten Parkinsonmitteln erreicht. Unter der erfindungsgemäßen Medikation wird auch eine Verkürzung der off-Phase erreicht.

Zur erfindungsgemäßen Verwendung kann das Co-Enzym oder sein physiologisch verträgliches Salz in üblicher Weise mit pharmazeutisch annehmbaren Hilfs- und Trägerstoffen konfektioniert werden. Gegebenenfalls kann Nicotinamid-adenindinucleotid auch in Kombination mit anderen Wirkstoffen, die zur Antiparkinsontherapie geeignet sind, verwendet werden - beispielsweise mit postsynaptischen Dopaminagonisten wie Lisurid oder Apomorphin oder Amantadin oder Deprenyl oder zentralen Anticholinergika (Y. Goodman and Gilman's: The Pharmacological Basis of Therapeutics, Chapter 21 Drugs for Parkinson's Disease, Spasticity and Acute Muscle Spasm, 7th Edition Macrhillan Publishing Company New York pp. 473-486, 1985).

Zur Verwendung als Arzneimittel kann Nicotinamid-adenin-dinucleotid in die üblichen galenischen Zubereitungsformen für die orale, parenterale (wie intravenöse oder subcutane) oder sublinguale Anwendung eingearbeitet werden. Die Herstellung der Arzneimittelspezialität erfolgt in an sich bekannter Weise, indem der Wirkstoff mit den in der galenischen Pharmazie gebräuchlichen Trägersubstanzen, Verdünnungsmitteln Geschmackskorrigentien usw. verarbeitet wird. Die Präparate können in fester Form als Tabletten, Kapseln, Dragees oder in flüssiger Form als Lösungen, Suspensionen, Sprays oder Emulsionen sowie Zubereitungsformen mit verzögerter Wirkstoffabgabe vorliegen. Zur Verbesserung der Motorik bei Parkinson Patienten beträgt die Einzeldosis bei parenteraler Applikation zwischen 5 bis 500 mg, vorzugsweise zwischen 25 bis 100 mg und die Tagesdosis zwischen 5 bis 1500 mg, vorzugsweise 25 bis 300 mg.

Die oralen Dosen können im gleichen Bereich wie die parenteral anzuwendenden Wirkstoffmengen liegen oder gegebenenfalls um 1000 % höher.

Liegt das Coenzym in Form seines physiologisch verträglichen Salzes vor, so sind hierfür alle bekannten sauren und basische Salzbildner geeignet wie anorganische Säuren wie z.B. Halogenwasserstoffsäuren, Schwefelsäure, Phosphorsäure oder organische Säuren wie aliphatische oder aromatische Carbonsäuren z.B. Ameisen-, Essig-, Bernstein-, Milch-, Apfel-, Wein-, Zitronen-, Malein-, Phenylessig-, Benzoe-, Salicylsäure; oder Ascorbinsäure; oder Alkali- oder Erdalkali-hydroxide oder -salze.

**Patentansprüche**

1.  Verwendung von Nicotinamid-adenin-dinucleotid oder eines Salzes davon, ausgenommen in einer Kombination mit Tyrosin, zur Herstellung eines zur Behandlung des Parkinson-Syndroms geeigneten Arzneimittels.

2.  Verwendung von Nicotinamid-adenin-dinucleotid gemäß Anspruch 1 in Kombination mit anderen Anti-Parkinsonmitteln.

**Claims**

1.  Use of nicotinamide adenine-dinucleotide or a salt thereof, except in a combination with tyrosine, for the production of a medicament suited for the treatment of Parkinson's syndrome.

2.  Use of nicotinamide-adenine dinucleotide in accordance with claim 1 in combination with other anti-Parkinson agents.

**Revendications**

1.  Application de nicotinamide adénine dinucléotide ou un de ses sels, à l'exception d'une combinaison avec la tyrosine, pour la préparation d'un médicament approprié pour le traitement de la maladie de Parkinson.

2.  Application de nicotinamide adénine dinucléotide selon la revendication 1 en combinaison avec d'autres agents anti-Parkinsonniens.